# EUROPEAN PATENT SPECIFICATION

(11) **EP 1 190 704 B1**
(45) Date of publication and mention of the grant of the patent: **18.01.2006**
(21) Application number: 00120643.2
(22) Date of filing: 21.09.2000
(51) Int. Cl.: A61K 8/41, A61K 8/46, A61Q 5/02, A61Q 5/12

(54) **Use of cosmetic compositions comprising anionic and cationic surfactants for the treatment of hair**
Verwendung von kosmetischen Zubereitungen enthaltend anionische und kationische Tenside zur Haarbehandlung
Utilisation de compositions cosmétiques comprenant des tensioactifs anioniques et cationiques pour le traitement des cheveux

(43) Date of publication of application: 27.03.2002
(73) Proprietor: Clariant S.A., 04795-900 Sao Paulo, SP (BR)
(72) Inventor: Coimbra, Luiz Fernando, Sao Paulo SP CEP 0670000 (BR); Gallotti, Manlio, 01421-01 Sao Paulo-SP (BR); Quartarolli, Simone Bergamo, Sao Paulo SP CEP 04134-040 (BR)
(74) Representative: Clariant Service GmbH

(56) References cited:
- EP-A- 0 384 715
- EP-A- 0 395 332
- WO-A-98/23720
- WO-A-98/59024
- WO-A-99/43773
- GB-A- 1 295 108
- GB-A- 2 292 155
- US-A- 4 235 759
- US-A- 5 714 446
- KARL HEINZ SCHRADER: "Grundlagen und Rezeptur der Kosmetika" 1989 , HÜTHIG BUCH VERLAG , HEIDELBERG XP002161527 * page 48 - page 49 *
- "Liquid Fabric Softeners" CLARIANT FUNCTIONAL CHEMICALS - MARKETS, [Online] 8 May 2000 (2000-05-08), XP002161526 Retrieved from the Internet: <URL:http://appmaster.clariant.com/html-su rfa/app_detergents/fabric_wash/liquid_fabr ic_soft.html > [retrieved on 2001-02-22]

## Description

The present invention relates to the use of cosmetic compositions for the treatment of hair comprising anionic and cationic surfactants.

Cosmetic compositions comprising anionic and cationic surfactants, i.e. surfactants which are considered to be non-compatible under normal conditions, often show low stability during storage because of precipitation and/or phase separation.

Applicants have now discovered that cosmetic compositions comprising at least one adduct formed by at least one anionic surfactant and at least one specific cationic surfactant are stable during storage.

The present invention therefore relates to the use of silicon-free cosmetic compositions comprising
a) from 0,1 % to 70 % by weight of at least one anionic surfactant;
b) from 0,05 % to 10 % by weight of at least one cationic surfactant selected from alkyl-dimethyl-hydroxyethyl-ammonium chloride wherein alkyl is selected from (C₈-C₂₂)-alkyl
c) optionally from 0.1 % to 15 % by weight of one or more nonionic and/or amphoteric surfactants for the treatment of hair.

Preferably the present compositions comprise from 2 to 50 %, highly preferred from 2 to 10 % by weight, of anionic surfactants a), from 0.5 to 8.0 %, highly preferred from 0.5 to 2.5 % by weight, of cationic surfactants b) and from 0.1 to 5 % by weight of nonionic and/or amphoteric surfactants c).

Preferred anionic surfactants are selected from the group consisting of alkyl sulfates, alkyl ether sulfates, secondary alkane sulfonates, α-olefin sulfonates, acyl isocyanates, linear alkylbenzene sulfonates, isethionates and mixtures thereof.

Said alkyl ether sulfates are preferably water-soluble salts or acids of the formula RO(A)ₘSO₃M, wherein R is an unsubstituted (C₁₀-C₂₄)-alkyl or (C₁₀-C₂₄)-hydroxyalkyl radical, preferably a (C₁₂-C₂₀)-alkyl or (C₁₂-C₂₀)-hydroxyalkyl radical, particularly preferred a (C₁₂-C₁₈)-alkyl or a (C₁₂-C₁₈)-hydroxyalkyl radical; A is an ethoxy or propoxy unit; m is a number greater than 0, preferably between 0.5 and 6, particularly preferred between 0.5 and 3; and M is a hydrogen atom or a cation, preferably a metal cation, like sodium, potassium, lithium, calcium or magnesium, an ammonium cation, a substituted ammonium cation, preferably a methylammonium-, dimethylammonium-, trimethylammonium-, mono-, di- or triethanolammonium cation, or a quaternary ammonium cation, preferably a tetramethylammonium cation, a dimethylpiperidinium cation or a cation derived from alkylamines like ethylamine, diethylamine or triethylamine.

Particularly preferred alkyl ether sulfates are (C₁₂-C₁₈)-alkyl-polyethoxylate (1.0) sulfate, (C₁₂-C₁₈)-alkyl polyethoxylate (2.25) sulfate, (C₁₂-C₁₈)-alkyl polyethoxylate (3.0) sulfate and (C₁₂-C₁₈)-alkyl polyethoxylate (4.0) sulfate.

Said secondary alkane sulfonates can contain a saturated or unsaturated alkyl group which can be linear or branched and which can optionally be substituted with a hydroxyl group. The sulfo group can be randomly distributed over the entire carbon chain. Preferred secondary alkane sulfonates contain linear alkyl chains having from 9 to 25 carbon atoms, preferably from 10 to 20 carbon atoms, particularly preferred from 13 to 17 carbon atoms. Preferred cations are sodium, potassium, ammonium, mono-, di- or triethanolammonium, calcium, magnesium or mixtures thereof. Particularly preferred is sodium.

Said α-olefin sulphonates preferably contain linear or branched (C₈-C₂₂)-alkyl or (C₈-C₂₂)-alkenyl groups. Preferred cations are sodium, potassium, ammonium, TEA, DEA, MEA, Triethylamine, magnesium or mixtures thereof.

Said alkylbenzene sulfonates preferably contain linear alkyl chains having from 9 to 25 carbon atoms, preferably from 10 to 13 carbon atoms. The alkyl group can either be saturated or unsaturated and can be branched or linear. Optionally the alkyl group can be substituted by a hydroxyl group. Preferred cations are sodium, potassium, ammonium, mono-, di- or triethanolammonium, calcium, magnesium and mixtures thereof.

Said nonionic and amphoteric surfactants are preferably selected from the group consisting of alkyl polyalkylene glycols, alkylaryl polyalkylene glycols, alkyldimethyl amine oxides, dialkyl methyl amine oxides, alkylamidopropyl amine oxides, alkyl glucamides, alkyl polyglycosides, oxalkylated fatty acids, oxalkylated fatty acid esters, alkyl amines, alkyl amidopropyl betaines, alkyl dimethyl betaines and alkyl amphoacetates or -diacetates. The alkyl groups may be partially or fully replaced by alkenyl groups and may be linear or branched. Said alkyl groups preferably contain 8 to 22 carbon atoms. The polyalkylene glycol groups preferably contain 1 to 20 ethoxy and/or propoxy units.

Highly preferred amphoteric surfactants are cocamidopropyl betaine, sodium lauroamphoacetate and disodium lauroamphodiacetate.

Suitable co-surfactants are e.g. acyl glutamates, amide ether sulfates, methyl taurides, sarcosynates, sodium laureth 13-carboxylate or mixtures thereof.

The compositions used in the present invention can optionally contain oils, emulsifers, thickeners, natural and synthetic polymers, stabilizers, waxes, fillers, acids , bases, buffers, biological active compounds, vitamines, natural extracts, solubilizers, UV-absorber, perfumes, perfume carriers, pearlescing agents, dyes, bleaching agents and/or other ingredients conventionally used in cosmetic compositions.

The cosmetic compositions can be liquids, emulsions, lotions, creams, gels, or solids. If solids they are preferably used in the form of bars. Advantageously the rheology of said compositions can be adjusted over a wide range by varying the weight rations of said anionic and cationic surfactants.

The cosmetic compositions are hair treatment compositions like shampoos, lotions, creams, sprays and lacquers, preferably shampoos, highly preferred shampoos for african-ethnic hair.
Advantageously such hair treatment compositions provide an improved ability to comb the hair. Moreover they allow to reduce or even to eliminate emollient agents and conditioners like propyleneglycols and esters. When applied to african-ethnic hair such hair treatment compositions advantageously provide a positive hair volume decrease.

The invention is illustrated in the following non limiting examples, in which all percentages are on a weight basis unless otherwise.

### Example 1: shampoo formulation

| | |
|---|---|
| Sodium Lauryl Ether Sulfate (*Genapol® LRO) | 7.85 |
| Alkyl Dimethyl Hydroxy Ethyl Ammonium Chloride (*Praepagen® HY) | 0.98 |
| Sodium Laureth 13-Carboxylate (*Sandopan® LS-24N) | 0.3 |
| Sodium Lauroamphoacetate (*Genagen® LAA) | 0.87 |
| Perfume | qs |
| Preserver | qs |
| Water | qs |
| NaCl | qs |

### Example 2: shampoo formulation

| | |
|---|---|
| α-Olefin Sulfonate (**Hostapur® OS) | 7.0 |
| Sodium Lauryl Ether Sulfate (*Genapol® LRO) | 3.0 |
| Sodium Lauroamphoacetate (*Genagen® LAA) | 4.0 |
| Alkyl Dimethyl Hydroxy Ethyl Ammonium Chloride (*Praepagen® HY) | 0.5 |
| Perfume | qs |
| Preserver | qs |
| Water | qs |
| NaCl | qs |

| | |
|---|---|
| (*commercialized by Clariant SA; **commercialized by Clariant GmbH). | |

## Claims

1. Use of a cosmetic silicone-free composition comprising
a) from 0,1 % to 70 % by weight of at least one anionic surfactant;
b) from 0,05 % to 10 % by weight of at least one cationic surfactant selected from alkyldimethyl-hydroxyethyl-ammonium chloride
wherein
alkyl is selected from (C₈-C₂₂)-alkyl and
c) optionally from 0.1 % to 15 % by weight of one or more nonionic and/or amphoteric surfactants
for the treatment of hair.

2. Use according to claim 1 **characterized in that** the cosmetic composition comprises from 2 % to 50 % by weight of anionic surfactants and from 0.5 % to 8.0 % by weight of cationic surfactants selected from alkyldimethyl-hydroxyethyl-ammonium chloride.

3. Use according to claim 1 or 2 **characterized in that** the anionic surfactants are selected from the group consisting of alkyl sulfates, alkyl ether sulfates, secondary alkane sulfonates, α-olefin sulfonates, acyl isocyanates, linear alkylbenzene sulfonates, isethionates and mixtures thereof.

4. Use according to anyone of claims 1 to 3 **characterized in that** the nonionic and amphoteric surfactants are selected from the group consisting of alkyl polyalkylene glycols, alkylaryl polyalkylene glycols, alkyldimethyl amine oxides, dialkyl methyl amine oxides, alkylamidopropyl amine oxides, alkyl glucamides, alkyl polyglycosides, oxalkylated fatty acids, oxalkylated fatty acid esters, alkyl amines, alkyl amidopropyl betaines, alkyl dimethyl betaines and alkyl amphoacetates or -diacetates.

5. Use according to claim 4 **characterized in that** the amphoteric surfactants are selected from the group consisting of cocamidopropyl betaine, sodium lauroamphoacetate and disodium lauroamphodiacetate.

6. Use according to anyone of claims 1 to 5 **characterized in that** the cosmetic composition additionally contains oils, emulsifiers, thickeners, natural and synthetic polymers, stabilizers, waxes, solubilizers, fillers, acids, bases, buffers, biological active compounds, vitamines, natural extracts, UV-absorber, perfumes, perfume carriers, pearlescing agents, dyes, bleaching agents and/or other ingredients conventionally used in cosmetic compositions.

7. Use according to anyone of claims 1 to 6 **characterized in that** the cosmetic composition is a shampoo.

8. Use according to claim 7 **characterized in that** the shampoo is a shampoo for african-ethnic hair.

## Patentansprüche

1. Verwendung einer silikonfreien kosmetischen Zubereitung, enthaltend:
a) von 0,1 Gewichts-% bis 70 Gewichts-% mindestens eines anionischen Tensides;
b) von 0,05 Gewichts-% bis 10 Gewichts-% mindestens eines kationischen Tensides, augewählt aus Alkyldimethyl-hydroxyethyl-ammonium-chlorid,
wobei
das Alkyl aus (C₈-C₂₂)-Alkyl ausgewählt wird, und
c) wahlweise von 0,1 Gewichts-% bis 15 Gewichts-% eines oder mehrerer nicht ionischer und/oder amphoterischer Tenside,
zur Haarbehandlung.

2. Verwendung nach Anspruch 1, **dadurch gekennzeichnet, dass** die kosmetische Zubereitung von 2 Gewichts-% bis 50 Gewichts-% anionische Tenside und von 0,5 Gewichts-% bis 8,0 Gewichts-% kationische Tenside, ausgewählt aus Alkyldimethyl-hydroxyethyl-ammonium-chlorid, enthält.

3. Verwendung nach Anspruch 1 oder 2, **dadurch gekennzeichnet, dass** die anionischen Tenside ausgewählt werden aus der Gruppe, bestehend aus Alkylsulfaten, Alklyethersulfaten, sekundären Alkansulfonaten, α-Olefinsulfonaten, Acylisocyanaten, linearen Akylbenzolsulfonaten, Isethionaten und deren Mischungen.

4. Verwendung nach einem der Ansprüche 1 bis 3, **dadurch gekennzeichnet, dass** die nicht ionischen and amphoterischen Tenside ausgewählt werden aus der Gruppe, bestehend aus Alkylpolyalkylenglykolen, Alkylarylpolyalkylenglykolen, Alkyldimethylaminoxiden, Dialkylmethylaminoxiden, Alkylamidopropylaminoxiden, Alkylglucamiden, Alkylpolyglykosiden, oxalkylierten Fettsäuren, oxalkylierten Fettsäureestern, Alkylaminen, Alkylamidopropylbetainen, Alkyldimethylbetainen and Alkylamphoacetaten oder -diacetaten.

5. Verwendung nach Anspruch 4, **dadurch gekennzeichnet, dass** die amphoterischen Tenside ausgewählt werden aus der Gruppe, bestehend aus Cocamidopropylbetain, Natriumlauroamphoacetat und Di-Natriumlauroamphodiacetat.

6. Verwendung nach einem der Ansprüche 1 bis 5, **dadurch gekennzeichnet, dass** die kosmetische Zubereitung zusätzlich Öle, Emulgatoren, Verdickungsmittel, natürliche and synthetische Polymere, Stabilisatoren, Wachse, Lösungsvermittler, Füllstoffe, Säuren, Basen, Puffer, biologisch aktive Verbindungen, Vitamine, natürliche Extrakte, UV-Absorber, Parfümstoffe, Parfüm-Träger, Perlglanzgeber, Farbstoffe, Bleichmittel und/oder sonstige üblicherweise in kosmetischen Zubereitungen verwendete Inhaltsstoffe enthält.

7. Verwendung nach einem der Ansprüche 1 bis 6, **dadurch gekennzeichnet, dass** die kosmetische Zubereitung ein Shampoo ist.

8. Verwendung nach Anspruch 7, **dadurch gekennzeichnet, dass** das Shampoo ein Shampoo für krauses Haar von Menschen afrikanischer Abstammung ist.

## Revendications

1. Utilisation, pour le traitement des cheveux, d'une composition cosmétique exempte de silicone, comprenant
a) de 0,1 à 70 % en poids d'au moins un tensioactif anionique ;
b) de 0,05 à 10 % en poids d'au moins un tensioactif cationique choisi parmi les chlorures d'alkyldiméthylhydroxyéthylammonium dans lesquels le groupe alkyle est choisi parmi les radicaux alkyle en C₈-C₂₂ ; et
c) en option de 0,1 à 15 % en poids d'un ou plusieurs tensioactifs non ioniques et/ou amphotères.

2. Utilisation selon la revendication 1, **caractérisée en ce que** la composition cosmétique comprend de 2 à 50 % en poids de tensioactifs anioniques, et de 0,5 à 8,0 % en poids de tensioactifs cationiques choisis parmi les chlorures d'alkyldiméthylhydroxyéthylammonium.

3. Utilisation selon la revendication 1 ou 2, **caractérisée en ce que** les tensioactifs anioniques sont choisis dans le groupe consistant en les alkylsulfates, les alkyléthersulfates, les alcanesulfonates secondaires, les α-oléfinesulfonates, les acylisocyanates, les (alkyle linéaire)benzènesulfonates, les iséthionates et leurs mélanges.

4. Utilisation selon l'une quelconque des revendications 1 à 3, **caractérisée en ce que** les tensioactifs non ioniques et amphotères sont choisis dans le groupe consistant en les alkylpolyalkylèneglycols, les alkylarylpolyalkylèneglycols, les oxydes d'alkyldiméthylamine, les oxydes de dialkylméthylamine, les oxydes d'alkylamidopropylamine, les alkylglucamides, les alkylpolyglycosides, les acides gras alcoxylés, les esters d'acides gras alcoxylés, les alkylamines, les alkylamidopropylbétaïnes, les alkyldiméthylbétaines et les alkylamphoacétates ou -diacétates.

5. Utilisation selon la revendication 4, **caractérisée en ce que** les tensioactifs amphotères sont choisis dans le groupe consistant en la coco-amidopropylbétaïne, le lauroamphoacétate de sodium et le lauroamphodiacétate disodique.

6. Utilisation selon l'une quelconque des revendications 1 à 5, **caractérisée en ce que** la composition cosmétique contient en outre des huiles, des émulsifiants, des épaississants, des polymères naturels et synthétiques, des stabilisants, des cires, des solubilisateurs, des matières de charge, des acides, des bases, des tampons, des composés biologiques actifs, des vitamines, des extraits naturels, des absorbants UV, des parfums, des supports de parfums, des agents nacrescents, des colorants, des agents de blanchiment et/ou d'autres ingrédients traditionnellement utilisés dans les compositions cosmétiques.

7. Utilisation selon l'une quelconque des revendications 1 à 6, **caractérisée en ce que** la composition cosmétique est un shampoing.

8. Utilisation selon la revendication 7, **caractérisée en ce que** le shampoing est un shampoing pour des cheveux africains-ethniques.
